# FASCICULE DE BREVET EUROPEEN

(11) **EP 2 039 389 B1**
(45) Date de publication et mention de la délivrance du brevet: **22.06.2011**
(21) Numéro de dépôt: 08290883.1
(22) Date de dépôt: 18.09.2008
(51) Int. Cl.: A61M 25/06, A61M 39/06

(54) **Nécessaire de mise en place d'une sonde intracorporelle, notamment d'une sonde de détection/stimulation cardiaque**
Implantationskit für eine Sonde zum Einsetzen in den Körper, insbesondere Sonde zur Erfassung/Stimulation des Herzschlags
Kit for implanting an intracorporeal probe, in particular a probe for cardiac detection/stimulation

(30) Priorité: 18.09.2007 FR 0706551
(43) Date de publication de la demande: 25.03.2009
(73) Titulaire: Ela Medical, 92541 Montrouge (FR)
(72) Inventeur: Shan, Nicolas, 94300 Vincennes (FR)
(74) Mandataire: Dupuis-Latour, Dominique

(56) Documents cités:
- EP-A- 1 002 553
- US-A- 4 512 766
- US-A1- 2005 010 238
- US-A1- 2006 167 417
- US-A1- 2007 123 825

## Description

L'invention concerne la mise en place des sondes intracorporelles, notamment les sondes de détection/stimulation cardiaque des "dispositifs médicaux implantables actifs" tels que définis par la directive 90/385/CEE du 20 juin 1990 du Conseil des communautés européennes, plus précisément des implants cardiaques de stimulation, de resynchronisation, de cardioversion et/ou de défibrillation.

Les sondes cardiaques peuvent être des sondes endocavitaires, comme les sondes de détection/stimulation des cavités droites, ou bien des sondes introduites dans le réseau coronarien, notamment les sondes comportant une électrode disposée face à une cavité gauche du myocarde.

L'introduction de ce dernier type de sonde, opérée par des voies endocavitaires, est une intervention particulièrement délicate compte tenu d'une part de la difficulté d'accès à l'entrée du sinus coronaire via l'oreillette droite, et d'autre part de la précision requise pour les points de stimulation une fois la sonde guidée et immobilisée dans le réseau coronarien.

L'une des techniques de mise en place d'une telle sonde met en oeuvre un accessoire dit "cathéter-guide". Cet accessoire comporte une gaine tubulaire creuse armée par un treillis métallique et dont la surface intérieure présente un faible coefficient de frottement (par exemple une surface de PTFE co-extrudée ou co-moulée avec le reste de la gaine). La flexibilité de la gaine est en outre étudiée de manière à présenter une rigidité en torsion suffisante pour permettre la transmission d'un mouvement de rotation d'une extrémité à l'autre, afin de pouvoir diriger l'extrémité de la sonde par rapport au myocarde lors de l'intervention.

Une fois posé, le cathéter-guide constitue un "tunnel" direct entre le "monde extérieur" et le sinus coronaire, tunnel que le chirurgien peut alors utiliser pour y glisser la sonde jusqu'à son emplacement définitif.

Une fois la sonde installée, le cathéter-guide doit être extrait, et cette manoeuvre est délicate car il faut bien entendu éviter de déloger la sonde ou modifier son positionnement et son orientation.

Une autre difficulté de cette étape tient à la présence du connecteur électrique situé à l'extrémité proximale de la sonde : le diamètre de ce connecteur étant supérieur à celui de la lumière interne du cathéter-guide, il empêche de retirer le cathéter-guide en le faisant simplement glisser vers l'arrière le long de la sonde.

L'étape d'extraction du cathéter-guide impose donc le plus souvent de le découper à son extrémité proximale le long d'une génératrice au moyen d'un outil à lame, dit *slitter*, qui vient fendre l'extrémité proximale du cathéter-guide et le treillis métallique formant l'armature de la gaine creuse. D'une main, le chirurgien tire alors le cathéter-guide vers lui d'un geste continu, en maintenant fermement de l'autre main la sonde et l'outil de découpe, qui vient simultanément fendre la gaine au fur et à mesure de son extraction.

On a certes proposé, pour éviter le recours à un outil tranchant, d'utiliser une gaine non armée, simplement pelable ; malheureusement, le prédécoupage de cette gaine sur toute sa longueur entraîne une faiblesse et une moindre rigidité du cathéter-guide, avec un risque de pliure et une moins bonne transmission des efforts lors de sa mise en place. Il a été également proposé, notamment par le EP-A-1 155 710 (ELA Medical) d'équiper la sonde d'un connecteur démontable, ce qui évite d'avoir à découper le cathéter-guide. Cette manière de procéder entraîne cependant une multiplication des étapes de mise en place et d'assemblage des divers éléments, compliquant d'autant l'intervention.

La découpe du cathéter-guide est donc, en pratique, la manière la plus courante de procéder.

Une difficulté supplémentaire de cette intervention tient à la présence, à l'extrémité proximale du cathéter-guide, d'une valve hémostatique permettant d'obturer à volonté la lumière interne débouchant à l'extrémité proximale, ouverte, du cathéter-guide.

Cette valve est généralement munie d'une voie latérale susceptible d'être mise en communication avec la lumière interne du cathéter-guide pour, d'une part, pouvoir purger le cathéter-guide après sa mise en place et, d'autre part, éventuellement y injecter à l'intérieur un agent antithrombotique ou un produit de contraste.

Cette valve hémostatique doit répondre à un certain nombre de contraintes, qui viennent s'ajouter aux difficultés exposées plus haut.

En premier lieu, la valve doit pouvoir être traversée par la sonde, afin de pouvoir faire pénétrer cette dernière dans le cathéter-guide puis la guider jusqu'à son emplacement définitif.

En second lieu, une fois la sonde mise en place, la valve doit pouvoir être dissociée du cathéter-guide, notamment pour permettre au chirurgien d'amorcer la découpe de la gaine du cathéter-guide afin d'extraire ce dernier.

Il a été proposé de nombreuses configurations d'ensembles cathéter-guide/valve hémostatique.

Dans le US-A-6 159 198 (Gardeski), la valve est une valve démontable, emboîtée par un montage de type *Luer lock* dans une tulipe de raccordement ou *hub* formée à l'extrémité proximale du cathéter-guide. La fermeture de la valve est assurée par un mécanisme vissant situé en partie arrière de la valve et permettant de ménager à volonté un passage vers la lumière interne afin d'y introduire la sonde. Pour permettre la découpe du cathéter-guide en fin d'intervention après démontage de la valve, la tulipe de raccordement est elle-même sécable (découpable) le long d'une partie amincie formée le long d'une génératrice et prolongée par une transition vers la gaine armée.

Le US-A-2005/0010238 (Potter) décrit une valve hémostatique montée en bout du cathéter-guide sur une tulipe de raccordement au moyen d'un système d'assemblage clipsé. A la différence de la précédente, la tulipe n'est pas sécable (c'est-à-dire impliquant le recours à un outil à lame pour la découper), mais frangible (c'est-à-dire qu'elle peut être cassée en deux sous l'effet d'un effort exercé par les doigts du chirurgien, sans outil additionnel). La tulipe de raccordement ayant été ainsi éliminée, l'extrémité proximale du cathéter-guide est directement accessible, permettant la découpe de celui-ci de la manière habituelle par un outil tranchant.

Le US-A-2004/0176781 (Lindstrom) décrit un nécessaire de mise en place d'une sonde pour un cathéter-guide qui n'est pas armé, mais simplement pelable, et ne nécessite donc pas d'outil de découpe pour son extraction. La procédure est toutefois rendue plus complexe par la nécessité de recourir à un accessoire supplémentaire dit *TVI* ou dilatateur, pour ouvrir l'élément d'obturation de la valve et protéger la sonde lors de la traversée de celle-ci. Le dilatateur a également pour fonction d'augmenter la rigidité de la gaine pelable, mais il doit bien entendu être lui aussi extrait après utilisation, ce qui nécessite une étape supplémentaire, le dilatateur devant par ailleurs lui aussi présenter une structure pelable.

Le US-A-6 966 996 (Kurth) décrit un nécessaire de mise en place d'une sonde dans lequel la valve, et non plus la tulipe, est frangible. Ceci permet de séparer en deux parties la valve, qui est initialement monobloc avec le cathéter-guide, pour laisser apparaître l'extrémité proximale de celui-ci et permettre sa découpe et son extraction.

Le US-A-2007/0123825 décrit un nécessaire mettant en oeuvre un introducteur pourvu d'une valve qui peut être dissociée en deux moitiés, qui sont ensuite écartées pour pouvoir peler l'introducteur (qui ne nécessite donc pas d'outil de découpe pour son retrait). La valve comprend en outre un moyen permettant, en pinçant les deux ailettes de la valve, d'ouvrir côté proximal un orifice axial découvrant la lumière d'une pièce axiale tubulaire prolongeant l'introducteur. Le cathéter muni de la sonde peut alors être introduit dans cet orifice de l'introducteur.

Les divers dispositifs proposés jusqu'à présent entraînent cependant tous un certain nombre de difficultés, en particulier :
- le risque est toujours grand, lors de l'intervention, de déplacer la sonde au moment de l'enlèvement de la valve et de l'extraction du cathéter-guide, en particulier avec les sondes pourvues d'un connecteur électrique de gros diamètre, car tous les accessoires du nécessaire d'introduction doivent être retirés en passant au-dessus de ce connecteur ;
- les valves manoeuvrées par un système à vis requièrent du chirurgien une doigté très précis pour éviter d'endommager la sonde en la serrant trop fort et, inversement, de créer un défaut d'étanchéité pendant l'intervention du fait d'un serrage insuffisant ;
- dans le cas d'une tulipe sécable, il est difficile de découper celle-ci avec un *slitter* standard, en raison de la brusque variation de l'effort de découpe lors de la transition entre le matériau de la tulipe et la gaine armée, avec un risque pour la sonde si le dosage de l'effort par le chirurgien n'est pas suffisamment précis ;
- par leur simplicité, les systèmes frangibles permettent une élimination aisée de la valve, mais présentent l'inconvénient de ne pas comprendre de moyen commode de manoeuvre de la valve, d'où le recours à un dilatateur pour introduire la sonde dans la valve ; cet accessoire "flotte" le long du conducteur de sonde pendant l'intervention et en tout état de cause devra être retiré par pelage en fin d'intervention ;
- les valves frangibles, qui sont réalisées monobloc avec le cathéter-guide, ne permettent pas de faire tourner la voie latérale autour du cathéter-guide pour le chirurgien ;
- tous les dispositifs proposés jusqu'à présent nécessitent un certain nombre d'accessoires ou éléments séparés, ce qui complique la tâche du chirurgien pendant l'opération critique de l'extraction du cathéter-guide, lorsque le chirurgien doit être concentré sur la position de la sonde ;
- dans la mesure où la sonde doit traverser ces divers accessoires, notamment le dilatateur, les électrodes de la sonde peuvent se trouver polluées, en particulier par le lubrifiant souvent utilisé avec la valve.

L'un des buts de l'invention est de remédier à l'ensemble de ces difficultés, en proposant un nécessaire comprenant une valve hémostatique et un cathéter-guide combinés, qui présente les avantages suivants :
- valve de type frangible, réduisant le risque de contact avec la sonde et de déplacement de celle-ci lors de l'élimination de la valve en fin d'intervention ;
- après élimination de la valve, obtention d'un cathéter-guide dont l'extrémité est libre de tout élément dans la direction de découpe de l'outil coupant, qui pourra être manipulé avec un effort constant pendant toute l'étape d'extraction du cathéter-guide ;
- manoeuvres simples et efficaces pour ouvrir et fermer la valve, par exemple par un simple mouvement de translation, sans recours à aucun outil d'insertion de type dilatateur ou autre, pour permettre notamment l'introduction libre et directe de la sonde dans la lumière du cathéter-guide ;
- insertion directe de la sonde dans le cathéter-guide, sans contact avec aucun autre élément, évitant ainsi tout risque de pollution de la sonde lors de son introduction et de sa traversée de la valve ;
- aucun accessoire nécessaire (autre que l'outil de découpe), que ce soit pour l'introduction de la sonde dans le cathéter-guide, pour l'enlèvement de la valve ou pour l'extraction du cathéter-guide ;
- possibilité pour la valve de tourner librement autour du cathéter-guide, de manière à pouvoir orienter la voie latérale dans n'importe quelle direction radiale par rapport au cathéter-guide maintenu fixe ;
- manipulation de type "tout ou rien" entre les positions ouverte et fermée de la valve, et sans risque de fuite ni d'endommagement de la sonde, à la différence notamment des systèmes à visser ou autres mécanismes nécessitant un doigté précis.

L'invention propose un nécessaire du type général tel que décrit dans le US-A-2007/0123825 précité, correspondant au préambule de la revendication 1. Pour atteindre les buts précités, l'invention comprend les éléments énoncés dans la partie caractérisante de la revendication 1.

Diverses caractéristiques subsidiaires avantageuses sont énoncées dans les sous-revendications.

On va maintenant décrire un exemple de mise en oeuvre de l'invention, en référence aux dessins annexés où les mêmes références numériques désignent d'une figure à l'autre des éléments identiques ou fonctionnellement semblables.

La figure 1 est une vue perspective de l'ensemble cathéter-guide/valve hémostatique de l'invention, en position fermée.

La figure 2 est une vue perspective de l'ensemble cathéter-guide/valve hémostatique de l'invention, en position ouverte.

La figure 3 illustre l'étape d'enlèvement de la valve après rupture de celle-ci, avec placement de l'outil de découpe face au cathéter-guide pour permettre l'incision de la gaine et l'extraction du cathéter-guide.

La figure 4 est une vue en coupe par un plan axial, montrant le détail de la structure interne de la valve hémostatique en position fermée et illustrant notamment la manière dont est assurée l'étanchéité à divers niveaux de cette valve.

La figure 5 est une vue partielle, en coupe par un plan axial, illustrant les mécanismes de butée de la valve en position fermée, et de verrouillage de la valve sur le cathéter-guide en position ouverte.

Les figures 6 et 7 sont des vues schématiques, en coupe par un plan axial, montrant la manière dont sont mis en communication les différents éléments du cathéter-guide et de la valve dans ses deux positions respectives fermée et ouverte.

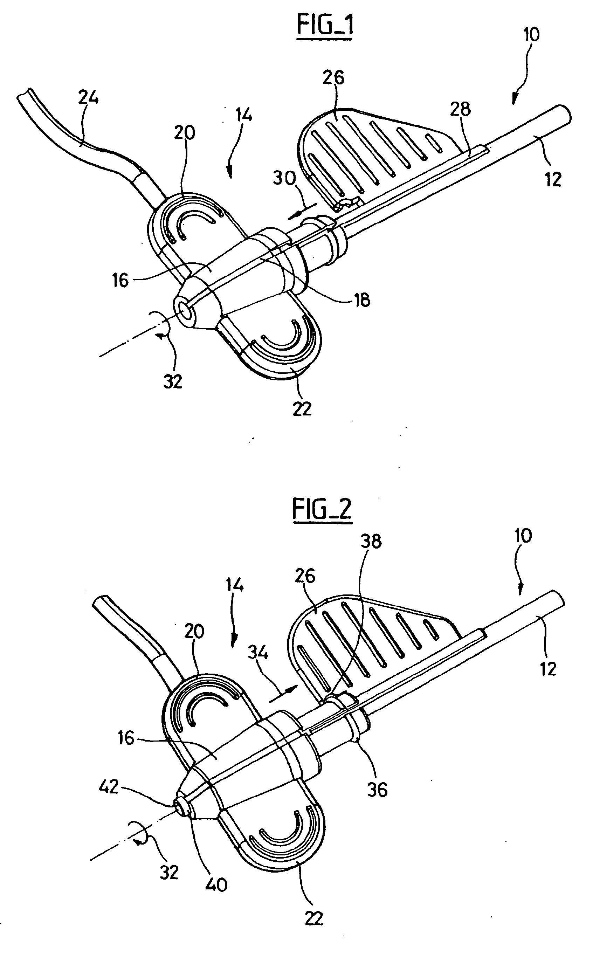

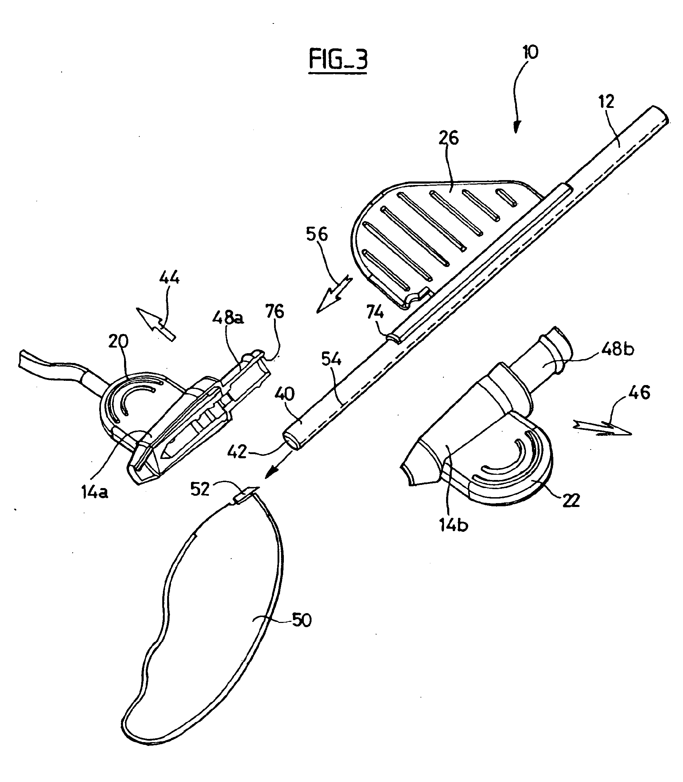

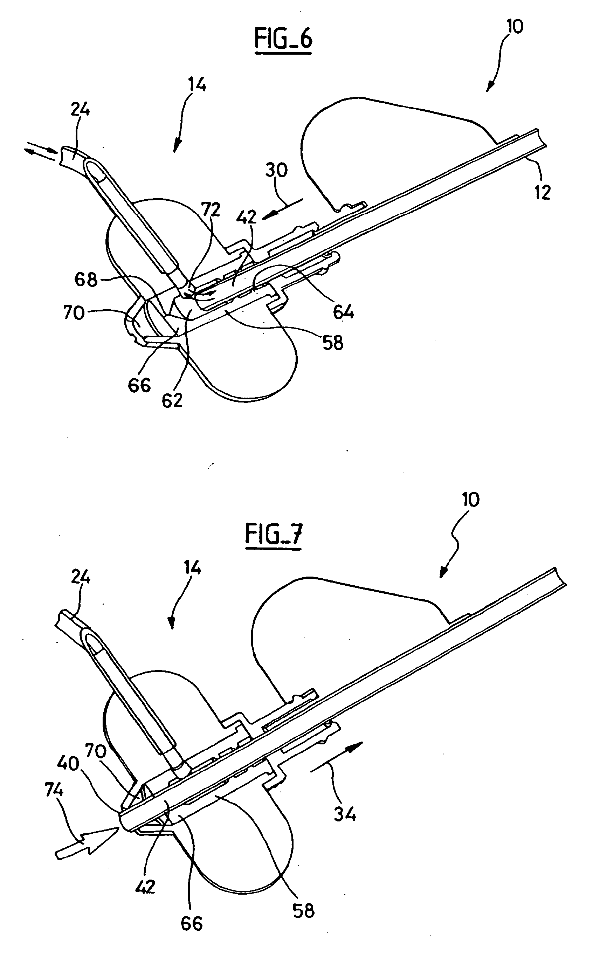

Les figures 1 à 7 illustrent différents aspects d'un même ensemble constitué d'un cathéter-guide et d'une valve hémostatique associée, la valve étant représentée selon le cas en position "fermée" (figures 1 et 7) ou "ouverte" (figures 2, 4, 5 et 6) ou encore après séparation de la valve d'avec le cathéter-guide, juste avant découpe de celui-ci (figure 3).

Le cathéter-guide 10 comprend une gaine creuse 12 armée par un treillis métallique pour en augmenter la rigidité ; une fois le cathéter-guide en place, son extrémité proximale (celle visible sur les différentes figures) émerge du site d'introduction, le chirurgien utilisant cette partie émergente pour introduire, diriger et positionner la sonde jusqu'à son emplacement définitif.

L'extrémité proximale du cathéter-guide 10 est pourvue d'une valve hémostatique 14 permettant d'obturer sélectivement la lumière interne de la gaine 12 à son extrémité débouchante.

De façon caractéristique de l'invention, cette valve 14 comporte un corps 16 mobile à coulissement sur le cathéter-guide entre deux positions extrêmes définissant l'état "ouvert" ou "fermé" de la valve, celle-ci étant manoeuvrée entre ces deux états par un mouvement très simple de type "aller-retour" ou *push-pull*.

Le corps 16 de la valve 14 comporte sur toute sa longueur, le long de deux génératrices diamétralement opposées, une région de moindre épaisseur 18 formant amorce de rupture et rendant ainsi la valve frangible, c'est-à-dire susceptible d'être cassée en deux parties dissociables de part et d'autre d'un plan axial médian contenant les amorces de rupture 18.

Pour faciliter sa manoeuvre, la valve est pourvue de deux ailettes latérales opposées 20, 22 s'étendant dans un plan axial. Ces ailettes permettent également, comme on le verra plus loin, d'exercer sur la valve un effort suffisant pour la briser en deux parties, lorsqu'il s'agira de l'éliminer en fin d'intervention.

De plus, de manière en elle-même connue, la valve est pourvue d'une voie latérale 24 susceptible d'être mise en communication avec la lumière interne du cathéter-guide pour en permettre la purge, et pour éventuellement injecter des produits de contraste ou un agent antithrombotique.

Pour faciliter la manipulation, le cathéter-guide est également pourvu d'une ailette de préhension 26 s'étendant dans un plan axial, et constituant par exemple le prolongement d'un renfort longitudinal 28, solidaire de la gaine 12 et s'étendant circonférentiellement sur une partie de celle-ci (de manière à en permettre ultérieurement la découpe sans être entravée par l'élément 28).

Il est aisé pour le chirurgien de manceuvrer la valve en tenant d'une main l'ailette 26 du cathéter-guide, et de l'autre main l'une des ailettes 20 ou 22 de la valve, par exemple en éloignant ces ailettes (flèche 30) pour placer la valve en position "fermée". On notera par ailleurs que la valve est librement mobile en rotation autour du cathéter-guide 10 (flèche 32), ce qui permet d'orienter la voie latérale 24 dans n'importe quelle direction procurant le moins de gêne possible au chirurgien.

Dans un mouvement opposé (flèche 34 sur la figure 2), le chirurgien placera la valve en position "ouverte" en rapprochant les ailettes 20, 22 de la valve et l'ailette 26 du cathéter-guide. La valve sera maintenue dans cette position par un système d'encliquetage utilisant un collet 36 formé sur le corps 16 de la valve dans la région la plus distale de celui-ci, ce collet 36 venant s'emboîter dans un évidement 38 formé dans l'ailette 26 du cathéter-guide.

On remarquera que la valve de l'invention procure au praticien une indication tactile immédiate de la position effective, ouverte ou fermée, à la différence notamment des valve à élément vissable. Il est par ailleurs possible d'ajouter un marquage visuel approprié pour rendre encore plus apparente cette indication de la position de la valve.

On notera par ailleurs que, dans la position ouverte, l'extrémité débouchante 40 de la gaine 12 du cathéter-guide10 émerge de la partie la plus proximale du corps 16 de la valve, donnant ainsi accès à la lumière interne 42 du cathéter-guide (voir figure 2) pour permettre notamment l'introduction de la sonde dans cette lumière.

Après positionnement de la sonde à son emplacement définitif, il y a lieu d'extraire le cathéter-guide, qui a fini de jouer son rôle, en prenant toutes les précautions requises pour ne pas déplacer la sonde pendant cette extraction.

La première étape consiste à retirer la valve pour pouvoir accéder directement à l'extrémité du cathéter.

Comme on l'a indiqué plus haut, la valve est une valve frangible, qui peut être cassée en deux parties 14a, 14b sensiblement symétriques (figure 3) de part et d'autre d'un plan axial médian, par un effort de flexion exercé par le chirurgien sur les deux ailettes opposées 20, 22 : les deux moitiés 14a, 14b peuvent alors être séparées et éloignées (flèches 44, 46) ce qui libère l'accès au cathéter-guide, qui n'est plus emboîté entre les deux moitiés 48a, 48b du corps de la valve.

Le cathéter-guide peut alors être découpé, de manière en elle-même connue, au moyen d'un outil tranchant ou *slitter* 50 comportant une lame 52 permettant de fendre la gaine armée du cathéter-guide. L'outil 50 est maintenu fixe d'une main par le chirurgien, et de l'autre main il tire vers lui le cathéter-guide, en s'aidant notamment de l'ailette 26, pour fendre la gaine 12 le long d'une génératrice 54. Le mouvement de découpe et de retrait du cathéter-guide est poursuivi jusqu'à extraction complète de celui-ci (flèche 56).

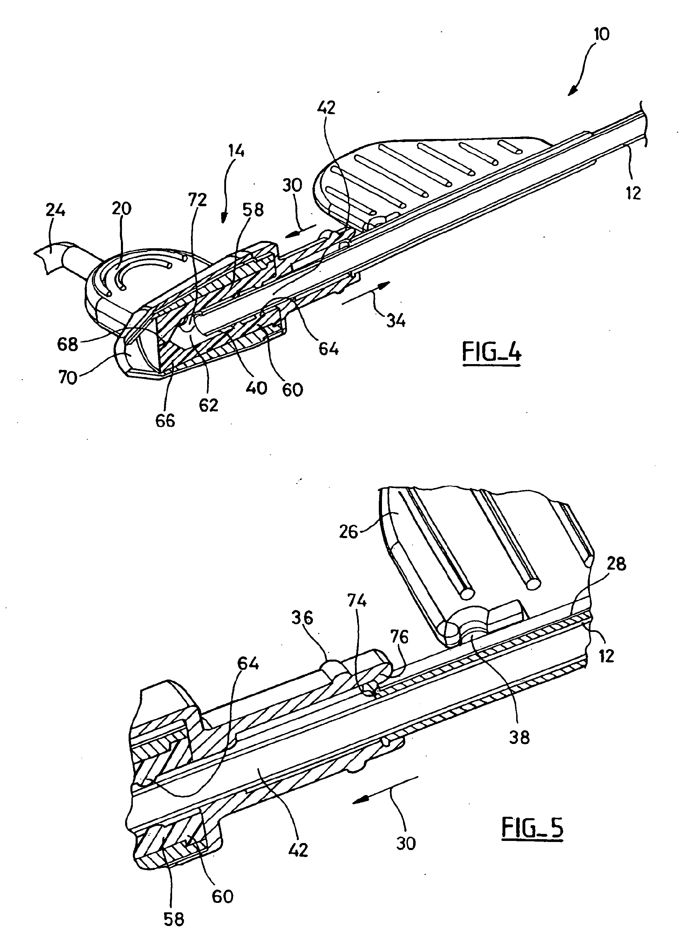

Les figures 4 à 7 sont des coupes illustrant la configuration interne des différents éléments de la valve 14, montée sur le cathéter-guide 10. Le corps de valve loge une pièce interne 58 en matériau élastiquement déformable, par exemple une silicone co-assemblée ou co-moulée avec le corps de la valve, qui est lui-même réalisé en un matériau rigide tel qu'un polypropylène ou un polyamide.

Dans sa région distale 60, la pièce élastique 58 définit une cavité centrale 62 de forme cylindrique, dans laquelle pénètre la gaine 12 du cathéter-guide 10. L'étanchéité entre la pièce élastique 58 et la gaine 12 est assurée par un ou plusieurs reliefs internes circonférentiels 64 obturant la cavité 62, et donc la lumière interne 42 du cathéter-guide, de manière étanche vis-à-vis de l'extérieur.

A son extrémité proximale 66, la pièce élastique 58 est fermée, c'est-à-dire que la cavité 62 est une cavité borgne ouverte seulement en direction distale. La paroi fermant la pièce élastique 58 constitue cependant un opercule traversable, grâce à un perçage ou une fente 68. En position libre, non sollicitée, du fait de l'élasticité du matériau de la pièce 58 cet opercule ferme de manière étanche la cavité 62, mais il peut être traversé sous l'effet d'une sollicitation extérieure par un élément enfoncé axialement, à savoir dans le cas présent l'extrémité 40 de la gaine du cathéter-guide 10 qui traverse l'opercule lorsque la valve est déplacée (flèche 34) vers la position d'ouverture) : comme illustré sur la figure 7, l'opercule est alors dilaté et traversé par l'extrémité 40 du cathéter-guide, qui émerge librement de la valve au travers d'un orifice 70 formé dans la région proximale du corps de valve.

Par ailleurs, la cavité interne 62 est munie d'un orifice radial 72 permettant de mettre en communication cette cavité 62, et donc la lumière interne 42 du cathéter-guide 10, avec la voie latérale 24 de la valve.

La figure 5 illustre plus en détail le mécanisme de butée et de verrouillage de la valve sur le cathéter-guide.

En premier lieu, lorsque la valve est sollicitée vers sa position fermée (flèche 30), une collerette 74, formée à l'extrémité de la pièce de renfort 28 solidaire du cathéter-guide, vient en butée contre un étranglement 76 formé à l'extrémité distale du corps de valve. Outre leur rôle de butée permettant de définir géométriquement la position "fermée" de la valve, ces éléments coopérants assurent la solidarisation mutuelle du cathéter-guide et de la valve, tout en ménageant un degré de liberté en rotation axiale de la valve autour du cathéter-guide, comme exposé plus haut. Le cathéter-guide ne pourra être séparé de le valve qu'en cassant celle-ci, comme on l'a décrit plus haut à propos de la figure 3, la collerette 74 du cathéter-guide n'étant alors plus retenue par l'étranglement 76 de la valve.

On a également illustré sur la figure 5 les éléments permettant de définir la position "ouverte" de la valve et le verrouillage dans cette position, grâce à un collet 36 de la valve venant s'encliqueter dans un évidement 38 de l'ailette 26 solidaire du cathéter-guide.

Les figures 4 et 6 illustrent les positions respectives du cathéter-guide et de la valve, respectivement dans les positions "fermée" et "ouverte" de celle-ci.

Comme on l'a indiqué plus haut, le passage d'une position à l'autre se fait simplement par une manipulation "aller-retour" ou *push-pull* de la valve par rapport au cathéter-guide (flèches 30, 34), cette manoeuvre étant de type "tout ou rien" et ne nécessitant aucune manipulation spécifique ou dosage d'effort, à la différence des mécanismes vissants utilisés dans l'art antérieur.

La manoeuvre facile et rapide de la valve entre les deux positions diminue en outre de façon importante les risques d'entrée d'air (et d'embolisme) ou de fuite de sang pendant l'intervention, contribuant ainsi de façon importante à la sécurité de la mise en place de la sonde.

En position ouverte (figure 7) on notera que l'extrémité proximale 40 du cathéter-guide émerge librement du corps de la valve, ce qui permet d'insérer la sonde directement dans le cathéter-guide, sans aucun risque de pollution de la sonde, car le cathéter-guide fait lui-même office de dilatateur pour ouvrir l'opercule 68 de la valve. Ceci évite en outre le recours à un outil spécifique, éliminant ainsi tous les inconvénients que l'on avait exposés au début de la description.

On notera enfin que l'émergence du cathéter-guide en partie proximale de la valve constitue une indication visuelle, simple et non ambiguë, de la position ouverte de la valve.

## Revendications

1. Un nécessaire de mise en place d'une sonde intracorporelle, notamment d'une sonde de détection/stimulation cardiaque, comprenant :
- un cathéter-guide (10), comportant une gaine (12) avec une lumière interne (42) ouverte à ses deux extrémités distale et proximale, cette gaine pouvant être ouverte le long d'une génératrice (54) pour permettre le retrait du cathéter-guide après usage ;
- une valve hémostatique (14) apte à sélectivement obturer, ou non, la lumière interne du cathéter-guide au débouché de celle-ci,
cette valve comportant un élément mobile par rapport au cathéter-guide entre deux positions extrêmes, avec une position ouverte où l'extrémité proximale (40) du cathéter-guide (10) émerge librement hors de la valve pour ménager un accès à la lumière interne du cathéter-guide, et une position fermée où cette extrémité proximale du cathéter-guide est obturée de manière étanche,
cette valve (14) étant une valve frangible en au moins deux parties (14a, 14b) dissociables entre elles de part et d'autre d'un plan axial médian,
- la gaine (12) du cathéter-guide (10) est une gaine, notamment une gaine armée rigidifiée, adaptée pour être découpée le long de ladite génératrice (54) au moyen d'un outil tranchant (50) comportant une lame (52) ; **caractérisé en ce que** :
- le cathéter-guide (10) présente un diamètre constant dans sa région recevant la valve, il est essentiellement dépourvu de tulipe de raccordement à son extrémité proximale, et il est emboîté entre lesdites au moins deux parties (14a, 14b) de la valve frangible (14), qui sont en outre dissociables chacune du cathéter-guide, de manière à pouvoir être séparées et éloignées afin de libérer l'accès au cathéter-guide ;
- la valve frangible (14) est montée à l'extrémité proximale (40) du cathéter-guide et comprend un corps (16) mobile à coulissement sur ce cathéter-guide entre lesdites positions extrêmes ouverte et fermée ; et
- le corps (16) de la valve comporte une cavité interne centrale (62) apte à recevoir l'extrémité proximale (40) du cathéter-guide (10), et comporte également du côté distal de ladite cavité interne (62) des moyens (64) d'étanchéité circonférentielle avec la gaine du cathéter-guide.

2. Le nécessaire de la revendication 1, dans lequel le corps (16) de la valve comporte du côté proximal de la cavité interne (62) un opercule (68) déformable entre, d'une part, une position non sollicitée où l'opercule obture ladite cavité interne du côté proximal et, d'autre part, une position distendue où l'opercule est traversé par la gaine du cathéter-guide en assurant une étanchéité circonférentielle avec celle-ci, la position non sollicitée correspondant à ladite position fermée, et la position distendue correspondant à ladite.position ouverte.

3. Le nécessaire de la revendication 2, dans lequel le corps (16) de la valve loge une pièce interne (58) en matériau élastiquement déformable, cette pièce interne incluant lesdits moyens d'étanchéité circonférentielle (64) côté distal et ledit opercule déformable (68) côté proximal.

4. Le nécessaire de la revendication 1, dans lequel la valve comprend une voie latérale (24) communiquant avec une dérivation radiale (72) de la cavité interne.

5. Le nécessaire de la revendication 1, dans lequel la valve est mobile librement en rotation axiale (32) autour du cathéter-guide.

6. Le nécessaire de la revendication 1, dans lequel la valve (14) comprend au moins une ailette de préhension (20, 22) s'étendant dans un plan axial.

7. Le nécessaire de la revendication 1, dans lequel le cathéter-guide (10) comprend au moins une ailette de préhension (26) s'étendant dans un plan axial.

8. Le nécessaire de la revendication 1, dans lequel la valve (14) comprend des moyens (36, 38) de verrouillage en position ouverte et/ou fermée.

9. Le nécessaire de la revendication 8, dans lequel lesdits moyens de verrouillage comprennent des moyens d'encliquetage coopérants (36, 38) prévus respectivement sur le corps (16) de la valve (14) et sur une extension axiale (26) du cathéter-guide (10).

## Claims

1. Kit for implanting an intracorporeal probe, notably a cardiac detection/stimulation probe, comprising:
- a catheter guide (10), comprising a sheath (12) with an internal opening (42) open at both its distal and proximal ends, this sheath being able to be opened along a generatrix (54) to allow the catheter guide to be removed after use;
- a haemostatic valve (14) capable of selectively blocking, or not, the internal opening of the catheter guide at its discharge end,
this valve comprising an element that is mobile relative to the catheter guide between two extreme positions, with an open position in which the proximal end (40) of the catheter guide (10) emerges freely from the valve to provide an access to the internal opening of the catheter guide, and a closed position in which this proximal end of the catheter guide is blocked in a leak-tight manner,
this valve (14) being a valve that is frangible into at least two mutually dissociable parts (14a, 14b) either side of a median axial plane,
- the sheath (12) of the catheter guide (10) is a sheath, notably a rigidified armoured sheath, suitable for being cut along said generatrix (54) by means of a cutting tool (50) comprising a blade (52);
**characterized in that**:
- the catheter guide (10) has a constant diameter in its region that receives the valve, it essentially has no connecting socket at its proximal end, and it is fitted between said at least two parts (14a, 14b) of the frangible valve (14), which are also each dissociable from the catheter guide, so as to be able to be separated and moved apart in order to free up access to the catheter guide;
- the frangible valve (14) is mounted at the proximal end (40) of the catheter guide and comprises a body (16) that is mobile sliding over this catheter guide between said open and closed extreme positions; and
- the body (16) of the valve includes a central internal cavity (62) capable of receiving the proximal end (40) of the catheter guide (10), and also includes, on the distal side of said internal cavity (62) sealing means (64) that are circumferential with the sheath of the catheter guide.

2. Kit according to Claim 1, in which the body (16) of the valve includes, on the proximal side of the internal cavity (62), a gate (68) that can be deformed between, on the one hand, an unstressed position in which the gate blocks said internal cavity on the proximal side and, on the other hand, a distended position in which the gate is passed through by the sheath of the catheter guide while ensuring a circumferential seal with the latter, the unstressed position corresponding to said closed position, and the distended position corresponding to said open position.

3. Kit according to Claim 2, in which the body (16) of the valve houses an internal part (58) made of elastically deformable material, this internal part including said circumferential sealing means (64) on the distal side and said deformable gate (68) on the proximal side.

4. Kit according to Claim 1, in which the valve comprises a lateral channel (24) communicating with a radial shunt (72) of the internal cavity.

5. Kit according to Claim 1, in which the valve is freely mobile in axial rotation (32) around the catheter guide.

6. Kit according to Claim 1, in which the valve (14) comprises at least one gripping fin (20, 22) extending in an axial plane.

7. Kit according to Claim 1, in which the catheter guide (10) comprises at least one gripping fin (26) extending in an axial plane.

8. Kit according to Claim 1, in which the valve (14) comprises means (36, 38) for locking in the open and/or closed position.

9. Kit according to Claim 8, in which said locking means comprise cooperating snap-fitting means (36, 38) provided respectively on the body (16) of the valve (14) and on an axial extension (26) of the catheter guide (10).

## Patentansprüche

1. Vorrichtung für die Anordnung einer intrakorporalen Sonde, insbesondere einer Detektions-/Stimulations-Herzsonde, die umfasst:
- eine Katheterführung (10), die eine Hülse (12) mit einem internen Langloch (42), das an seinem distalen und an seinem proximalen Ende offen ist, aufweist, wobei diese Hülse längs einer Erzeugenden (54) offen sein kann, um das Zurückziehen der Katheterführung nach der Verwendung zu ermöglichen;
- ein hämostatisches Ventil (14), das das interne Langloch der Katheterführung am Ausgang desselben wahlweise verschließen kann oder nicht,
wobei dieses Ventil ein Element umfasst, das in Bezug auf die Katheterführung zwischen zwei Extrempositionen, nämlich zwischen einer offenen Position, in der das proximale Ende (40) der Katheterführung (10) frei aus dem Ventil vorsteht, um in dem internen Langloch der Katheterführung einen Zugang auszubilden, und einer geschlossenen Position, in der dieses proximale Ende der Katheterführung dicht verschlossen ist, beweglich ist,
wobei dieses Ventil (14) ein in wenigstens zwei Teile (14a, 14b) zerbrechliches Ventil ist, die beiderseits einer mittleren axialen Ebene voneinander getrennt werden können,
- wobei die Hülse (12) der Katheterführung (10) eine Hülse, insbesondere eine versteifte verstärkte Hülse ist, die dazu ausgelegt ist, längs der Erzeugenden (54) mittels eines eine Klinge (52) aufweisenden Schneidwerkzeugs (50) zerschnitten zu werden;
**dadurch gekennzeichnet, dass**
- die Katheterführung (10) in ihrem das Ventil aufnehmenden Bereich einen konstanten Durchmesser aufweist, dass sie an ihrem proximalen Ende im Wesentlichen keine Verbindungserweiterung aufweist und dass sie zwischen die wenigstens zwei Teile (14a, 14b) des zerbrechlichen Ventils (14) eingesetzt ist, die außerdem jeweils von der Katheterführung getrennt werden können, so dass sie voneinander getrennt und entfernt werden können, um den Zugang zu der Katheterführung freizugeben;
- wobei das zerbrechliche Ventil (14) am proximalen Ende (40) der Katheterführung montiert ist und einen Körper (16) aufweist, der an dieser Katheterführung zwischen der offenen und der geschlossenen Extremposition gleitend beweglich ist; und
- der Körper (16) des Ventils einen mittigen inneren Hohlraum (62) aufweist, der das proximale Ende (40) der Katheterführung (10) aufnehmen kann und außerdem auf der distalen Seite des inneren Hohlraums (62) Mittel (64) für eine Umfangsdichtung mit der Hülse der Katheterführung umfasst.

2. Vorrichtung nach Anspruch 1, wobei der Körper (16) des Ventils auf der proximalen Seite des inneren Hohlraums (62) ein Verschlussorgan (68) aufweist, das einerseits zwischen einer nicht belasteten Position, in der das Verschlussorgan den inneren Hohlraum auf der proximalen Seite verschließt, und andererseits einer entspannten Position, in der das Verschlussorgan von der Hülse der Katheterführung durchquert wird und die Umfangsdichtigkeit mit dieser sicherstellt, verformbar ist, wobei die nicht belastete Position der geschlossenen Position entspricht und die entspannte Position der offenen Position entspricht.

3. Vorrichtung nach Anspruch 2, wobei der Körper (16) des Ventils ein Innenteil (58) aus einem elastisch verformbaren Material aufweist, wobei dieses Innenteil Umfangsdichtungsmittel (64) auf der distalen Seite und das verformbare Verschlussorgan (68) auf der proximalen Seite enthält.

4. Vorrichtung nach Anspruch 1, wobei das Ventil einen seitlichen Weg (24) aufweist, der mit einer radialen Abzweigung (72) des Innenhohlraums kommuniziert.

5. Vorrichtung nach Anspruch 1, wobei das Ventil axial rotatorisch um die Katheterführung frei beweglich ist.

6. Vorrichtung nach Anspruch 1, wobei das Ventil (14) wenigstens einen Griffsteg (20, 22) aufweist, der sich in einer axialen Ebene erstreckt.

7. Vorrichtung nach Anspruch 1, wobei die Katheterführung (10) wenigstens einen Griffsteg (26) aufweist, der sich in einer axialen Ebene erstreckt.

8. Vorrichtung nach Anspruch 1, wobei das Ventil (14) Mittel (36, 38) für die Verriegelung in der geöffneten und/oder geschlossenen Position umfasst.

9. Vorrichtung nach Anspruch 8, wobei die Verriegelungsmittel zusammenwirkende Einrastmittel (36, 38) umfassen, die am Körper (16) des Ventils (14) bzw. an einer axialen Verlängerung (26) der Katheterführung (10) vorgesehen sind.
